# EUROPÄISCHE PATENTSCHRIFT

(11) **EP 2 488 861 B1**
(45) Veröffentlichungstag und Bekanntmachung des Hinweises auf die Patenterteilung: **30.05.2018**
(21) Anmeldenummer: 10768737.8
(22) Anmeldetag: 13.10.2010
(51) Int. Cl.: G01N 33/487

(54) **SCHUTZ VOR HYDROPHOBIERENDEN AGENZIEN**
PROTECTION FROM HYDROPHOBIC AGENTS
PROTECTION CONTRE DES AGENTS HYDROPHOBES

(30) Priorität: 15.10.2009 EP 09173175
(43) Veröffentlichungstag der Anmeldung: 22.08.2012
(73) Patentinhaber: F. Hoffmann-La Roche AG, 4070 Basel (CH); Roche Diabetes Care GmbH, 68305 Mannheim (DE)
(72) Erfinder: BABIC, Bransilav, 68167 Mannheim (DE); LEICHNER, Wilhelm, 68307 Mannheim (DE)
(74) Vertreter: Weickmann & Weickmann PartmbB
(86) Internationale Anmeldenummer: PCT/EP2010/065359
(87) Internationale Veröffentlichungsnummer: WO 2011/045348

(56) Entgegenhaltungen:
- EP-A- 0 640 393
- EP-A- 1 884 188
- EP-A1- 2 093 162
- DE-A1-102004 033 317

## Beschreibung

Die vorliegende Erfindung betrifft Vorratsbehälter zur Lagerung diagnostischer Elemente mit hydrophiler oder hydrophil beschichteter Oberfläche. Weiterhin betrifft die Erfindung analytische Messgeräte, welche derartige Vorratsbehälter umfassen, sowie die Verwendung eines Absorptionsmaterials zur selektiven Absorption hydrophober, flüchtiger Substanzen in solchen Vorratsbehältern.

Diagnostische Elemente sind wichtige Bestandteile klinisch relevanter Analyseverfahren. Hierbei steht die Messung von Analyten, z.B. Metaboliten oder Substraten, im Vordergrund, welche beispielsweise mit Hilfe eines für den Analyten spezifischen Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym und einem Coenzym in Kontakt gebracht, wobei das Enzym meist in katalytischen Mengen eingesetzt wird. Das Coenzym wird durch die enzymatische Reaktion physikochemisch verändert, z.B. oxidiert bzw. reduziert, und der Vorgang beispielsweise elektrochemisch oder photometrisch erfasst. Eine Kalibrierung liefert einen direkten Zusammenhang des Messwerts mit der Konzentration des zu bestimmenden Analyten.

Ein wichtiges Kriterium bei der Bereitstellung diagnostischer Elemente ist deren Fähigkeit, eine den Analyten enthaltende Probe aufnehmen zu können. Zu diesem Zweck umfassen zahlreiche kommerziell erhältliche diagnostische Elemente eine hydrophile oder hydrophil beschichtete Oberfläche, welche bei Inkontaktbringen des diagnostischen Elements mit einer den Analyten enthaltenden flüssigen Probe eine Benetzung des Testelements mit der Probe sowie deren Aufnahme in das Testelement, beispielsweise mittels eines Probennahmeelements und unter Ausnutzung von Kapillareffekten, ermöglicht.

Als Problem erweist sich bei oben beschriebenen diagnostischen Elementen häufig deren Langzeitstabilität. So ist bekannt, dass sich frisch aus dem Ätzbad stammende metallische Probennahmeelemente, welche eine hydrophile Oberfläche aufweisen, schnell mit wässrigen Flüssigkeiten wie beispielsweise Blut füllen. Nach kurzer Zeit an der Luft oder nach kurzer Lagerung in üblichen Verpackungen verlängert sich die Füllzeit des Probennahmeelements indessen zunehmend und resultiert schließlich in einem vollständigen Verlust der Fähigkeit zur Probenaufnahme. Durch Plasmabehandlung dieser nicht-funktionellen Probennahmeelemente lässt sich die Füllzeit wieder auf das ursprüngliche Niveau verkürzen; allerdings wird innerhalb weniger Tage ein erneuter Verlust der Fähigkeit zur Probenaufnahme festgestellt.

Der zunehmende Verlust der Fähigkeit von Probennahmeelementen, eine Probe des zu bestimmenden Analyten aufzunehmen, beruht auf einer fortschreitenden Hydrophobierung der im ursprünglichen Zustand hydrophilen oder hydrophil beschichteten Oberfläche der Probennahmeelemente, welche durch hydrophobe Substanzen, wie beispielsweise hydrophobe, mäßig flüchtige organisch chemische Substanzen, die mit dem Probennahmeelement in Kontakt treten, bewirkt wird.

Als besonders kritisch haben sich in diesem Zusammenhang Verpackungen für diagnostische Elemente erwiesen, welche zumindest teilweise aus Kunststoff bestehen und infolge langsamen Austretens niedermolekularer, hydrophober Verbindungen aus dem Kunststoff, wie beispielsweise von niedrig siedenden Lösemitteln oder Restmonomeren, für eine signifikante Hydrophobierung hydrophiler Oberflächen verantwortlich sind. Neben diesen aus Verpackungsmaterialien austretenden Substanzen können allerdings auch hydrophobe, flüchtige Substanzen, welche bei Herstellung der zu lagernden diagnostischen Elemente Anwendung finden oder hierbei gebildet werden und beispielsweise in der Chemiebeschichtung der Testelemente enthalten sind, eine Hydrophobierung hydrophil beschichteter Oberflächen bewirken und damit zur Unbrauchbarkeit von Probennahmeelementen führen, die zusammen mit dem diagnostischen Element verpackt werden.

Sofern die diagnostischen Elemente vor Gebrauch sterilisiert werden müssen, stellt sich das zusätzliche Problem, dass beispielsweise bei Bestrahlen eines bereits verpackten diagnostischen Elements mit ionisierender Strahlung im allgemeinen eine Schädigung der Verpackung oder/und des diagnostischen Elements bewirkt wird, in deren Folge wiederum hydrophobe, flüchtige Substanzen erzeugt und freigesetzt werden können, welche zur Hydrophobierung eines in der Verpackung gelagerten, hydrophil beschichteten Probennahmeelements geeignet sind.

Um eine Hydrophobierung von Gegenständen mit hydrophiler Oberfläche durch das Verpackungsmaterial zu vermeiden und dadurch eine hohe Hydrophilie der Oberfläche über lange Lagerzeiträume zu gewährleisten, schlägt WO 2008/015227 A1 die Verwendung einer Verpackung vor, welche in ihrem Inneren mindestens eine lose Abdeckung oder/und mindestens eine adsorbierende Oberfläche umfasst, deren Affinität für apolare Stoffe gleich groß oder größer ist als diejenige der hydrophilen Oberfläche des zu lagernden Gegenstandes.

Im Einzelnen sieht vorstehendes Dokument die Verwendung von Adsorberelementen bzw. Adsorberschichten in Form einer hydrophilen Beschichtung vor, deren Hydrophilie bzw. Oberflächenenergie gleich oder größer ist als die Hydrophilie bzw. Oberflächenenergie der Oberfläche des verpackten Gegenstands, um eine effektive Adsorption der apolaren Gase an die Adsorberschicht sicherzustellen. Dabei schlagen sich die apolaren Gase u.a. auf der hydrophilen Oberfläche des Adsorberelements oder der Adsorberschicht nieder, wodurch die hydrophile Oberfläche des verpackten Gegenstandes zumindest teilweise vor einer Hydrophobierung geschützt wird. Materialien, welche für derartige hydrophile Beschichtungen geeignet sind, umfassen beispielsweise Dextransulfat, Lecithin, Polyacrylsäuren und Polyacrylate.

Die in WO 2008/015227 A1 zur Adsorption apolarer Gase eingesetzten hydrophilen Beschichtungen sind allerdings mit Nachteilen behaftet. So gewährleisten die in obiger Druckschrift beschriebenen Verpackungen de facto keine selektive Adsorption hydrophober Substanzen, wodurch letztlich auch kein ausreichender Schutz vor einer Hydrophobierung der hydrophilen Oberfläche eines in der Verpackung zu lagernden Gegenstandes erzielt werden kann.

In diesem Zusammenhang stellt sich zum einen das Problem, dass auch die hydrophile Oberfläche des in der Verpackung zu lagernden Gegenstandes mehr oder weniger stark hydrophobiert wird, wenn die Affinität der adsorbierenden Oberfläche für apolare Stoffe gleich groß oder zumindest nicht signifikant größer ist als diejenige der hydrophilen Oberfläche des zu lagernden Gegenstandes. Folglich adsorbieren die apolaren Gase unter derartigen Bedingungen nicht bevorzugt oder ausschließlich an die hydrophile Oberfläche des Adsorberelements bzw. der Adsorberschicht.

Insbesondere besitzen die in WO 2008/015227 A1 beschriebenen hydrophilen Beschichtungen jedoch den Nachteil, dass sie aufgrund ihrer hohen Hydrophilie bevorzugt Wasser (und nicht apolare Gase) adsorbieren. Sofern die Oberfläche des Adsorberelements bzw. der Adsorberschicht eine höhere Hydrophilie besitzt als die Oberfläche des verpackten Gegenstandes, so weist sie auch eine höhere Affinität für Wasser auf als die Oberfläche des verpackten Gegenstandes.

Dies führt bei hoher Luftfeuchtigkeit letztlich dazu, dass die Oberfläche des Adsorberelements bzw. der Adsorberschicht mit Wassermolekülen abgesättigt ist und Ausdunstungen aus dem Verpackungsmaterial nicht mehr von dem Adsorberelement bzw. von der Adsorberschicht aufgenommen werden können, womit es zu Ablagerungen auf der hydrophilen Oberfläche des verpackten Gegenstandes und damit zu deren Hydrophobierung kommt.

Die der Erfindung zugrunde liegende Aufgabe bestand somit darin, einen Vorratsbehälter zur Lagerung diagnostischer Elemente mit hydrophiler oder hydrophil beschichteter Oberfläche bereitzustellen, bei welchem die Nachteile des Standes der Technik zumindest teilweise beseitigt sind. Insbesondere sollte das diagnostische Element über einen längeren Zeitraum gelagert werden können, ohne hierbei einen Verlust der Hydrophilie seiner Oberfläche zu erleiden.

Diese Aufgabe wurde erfindungsgemäß gelöst mittels eines Vorratsbehälters, umfassend
(a) mindestens ein diagnostisches Element mit hydrophiler oder hydrophil beschichteter Oberfläche, und
(b) mindestens ein Absorptionsmaterial zur selektiven Absorption hydrophober, flüchtiger Substanzen.

Erfindungsgemäß enthält der Vorratsbehälter mindestens ein Absorptionsmaterial, welches einer selektiven Absorption hydrophober, flüchtiger Substanzen dient. Das Absorptionsmaterial, welches in Vorratsbehältern der vorliegenden Erfindung Anwendung findet, kann anorganischer oder organischer Natur sein und umfasst ein beliebiges Material, welches eine selektive Absorption hydrophober, flüchtiger Substanzen ermöglicht. Beispiele für derartige Absorptionsmaterialien umfassen u.a. Aktivkohle, Controlled Porous Glass (CPG), Kieselgele, Silikate und Alumosilikate (auch Aluminosilikate genannt), welche vornehmlich aus der Gruppe bestehend aus Gerüstsilikaten und Schichtsilikaten ausgewählt sind.

Der Ausdruck "selektive Absorption hydrophober, flüchtiger Substanzen", wie er in vorliegender Anmeldung verwendet wird, bedeutet, dass das Absorptionsmaterial bei den jeweiligen Umgebungsbedingungen, d. h. bei dem jeweils vorherrschenden Druck, der jeweils vorherrschenden Temperatur und der jeweils vorherrschenden relativen Luftfeuchtigkeit, eine höhere Affinität für mindestens eine hydrophobe, flüchtige Substanz im Sinne der vorliegenden Anmeldung aufweist als für Wasser.

Infolge Verwendung eines Absorptionsmaterials, welches eine selektive Absorption hydrophober, flüchtiger Substanzen gestattet, kann eine gezielte Abtrennung der hydrophoben, flüchtigen Substanz(en) von gleichzeitig in der Umgebung vorhandenem Wasserdampf erreicht werden. Dies bedeutet andererseits, dass das erfindungsgemäß eingesetzte Absorptionsmaterial nicht durch vorrangige Bindung von in der Umgebung vorhandenem Wasserdampf inaktiviert werden kann, bevor es mindestens eine, in der Umgebung gleichzeitig vorhandene hydrophobe, flüchtige Substanz aufgenommen hat.

Sofern das Absorptionsmaterial Poren aufweist, ist die Porengröße des oben beschriebenen Absorptionsmaterials derart ausgestaltet, dass in Gegenwart eines Gemisches aus mindestens einer hydrophoben, flüchtigen Substanz und Wasserdampf bevorzugt die mindestens eine hydrophobe, flüchtige Substanz in das Absorptionsmaterial eingelagert wird, so dass auch bei einer hohen relativen Luftfeuchtigkeit keine Sättigung des Absorptionsmaterials infolge Einlagerung von Wasser erfolgt. Gelangt ein porenhaltiges Absorptionsmaterial zur Anwendung, so hat dies gegenüber den in WO 2008/015227 A1 beschriebenen Adsorberelementen bzw. Adsorberschichten, welche apolare Gase oder/und Wasserdampf jeweils lediglich an ihrer Oberfläche binden, zudem den Vorteil, dass infolge der Poren die Aufnahmekapazität des Absorptionsmaterials für hydrophobe, flüchtige Substanzen deutlich erhöht werden kann.

Der Begriff "hydrophobe Substanz", wie er im Rahmen der vorliegenden Anmeldung verwendet wird, umfasst jegliche Substanz, welche aufgrund ihrer chemischen Struktur die Tendenz zeigt, in Wasser nicht einzudringen oder eine wässrige Phase zu verlassen. Die Hydrophobie ist dabei überwiegend an apolare Gruppen, wie beispielsweise aromatische Gruppen oder langkettige Kohlenwasserstoffreste, geknüpft, welche den Effekt gegebenenfalls zusätzlich vorhandener hydrophiler Gruppen überwiegen.

Der Begriff "flüchtige Substanz", wie er im Rahmen der vorliegenden Anmeldung verwendet wird, bedeutet, dass die betreffende Substanz bei einer Temperatur von 20°C einen Dampfdruck von ≥ 0.1 kPa aufweist. Typische Vertreter für flüchtige Substanzen im Sinne der vorliegenden Anmeldung sind hydrophobe, organisch chemische Substanzen mit einem Molekulargewicht zwischen 100 und 1000 Dalton.

Als vorteilhaft hat sich im Rahmen der Erfindung die Verwendung eines natürlich vorkommenden oder synthetischen Silikats als Absorptionsmaterial erwiesen. Der Begriff "synthetisches Silikat", wie er im Rahmen der vorliegenden Erfindung verwendet wird, umfasst sowohl vollsynthetische Silikate als auch Silikate, welche durch künstliche Veränderung (z. B. chemisch) eines natürlich vorkommenden Silikats erhalten werden. Beispiele für natürlich vorkommende oder synthetische Silikate umfassen Feldspäte, Glimmer, Mullit, Sillimanit und Zeolithe, sind jedoch nicht auf diese beschränkt.

Stärker bevorzugt handelt es sich bei dem Absorptionsmaterial um ein natürlich vorkommendes oder synthetisches Zeolith, in welchem Polyeder, Schichten oder Ketten aus eckenverknüpften [(Al,Si)O₄]-Tetraedern vorliegen, die ein von Kanälen durchzogenes, anionisches Raumnetzwerk mit regelmäßig angeordneten Hohlräumen ausbilden. Zeolithe, welche für die Zwecke der vorliegenden Erfindung geeignet sind, können vom Fachmann entsprechend den jeweiligen Anforderungen ausgewählt werden und umfassen insbesondere, ohne hierauf beschränkt zu sein, mittel- und weitporige Zeolithe, wie die unter der Handelsbezeichnung ZEOflair® 100, ZEOflair® 200 und ZEOflair® 300 (Fa. Zeochem, Schweiz) kommerziell erhältlichen Zeolithe. Das Absorptionsmaterial weist Poren mit einem Durchmesser von etwa 0.5 nm bis etwa 0.8 nm auf, in welchen eine Absorption der hydrophoben, flüchtigen Substanzen erfolgen kann. Vorzugsweise besitzen die Poren des Absorptionsmaterials eine kanalartige oder tunnelartige Struktur, wodurch eine große effektive Oberfläche bereitgestellt wird, die mit den zu absorbierenden Substanzen in Kontakt treten kann.
Erfindungsgemäß kann der in vorliegender Anmeldung beschriebene Vorratsbehälter das Absorptionsmaterial grundsätzlich in beliebiger Form enthalten, sofern auf diese Weise eine selektive Absorption hydrophober, flüchtiger Substanzen ermöglicht wird. Vorzugsweise enthält der Vorratsbehälter das mindestens eine Absorptionsmaterial in Form eines separaten Absorberelements oder/und in seinem Gehäuse integriert, wobei als separates Absorberelement im Sinne der vorliegenden Erfindung beispielsweise eine flache Scheibe oder ein beliebig geformter Block dienen kann.
Sofern der Vorratsbehälter das Absorptionsmaterial in seinem Gehäuse integriert enthält, bedeutet dies, dass das Absorptionsmaterial in das feste Gehäuse des Vorratsbehälters eingebracht ist und mindestens eine innere Oberfläche des Vorratsbehälters eine Aufnahme hydrophober, flüchtiger Substanzen ermöglicht. Derartige Vorratsbehälter können beispielsweise durch Einspritzen des mindestens einen Absorptionsmaterials in eine zur Herstellung des Gehäuses verwendete Form und anschließendes Abkühlen/Aushärten der plastischen Zusammensetzung erzeugt werden. Die Größe der Partikel des Absorptionsmaterials kann entsprechend den jeweiligen Anforderungen variiert werden. Erfindungsgemäß können im Falle einer separaten Zugabe des Absorptionsmaterials in den Innenraum des Vorratsbehälters beispielsweise grobkörnige Partikel mit einer Größe von bis zu mehreren Millimetern, d. h. mit einer Größe von bis zu etwa 5 mm, verwendet werden. Für die Einmischung des Absorptionsmaterials in das Gehäuse des Vorratsbehälters, z. B. in ein durch nachfolgendes Spritzgießen zu verarbeitendes Kunststoffmaterial, werden hingegen vorzugsweise möglichst feine Partikel mit einer Größe im Bereich von ≤ 1 µm eingesetzt.

In einer weiteren bevorzugten Ausführungsform enthält der erfindungsgemäße Vorratsbehälter mehr als ein Absorptionsmaterial, d. h. mindestens zwei unterschiedliche Absorptionsmaterialien, welche sich sowohl hinsichtlich ihrer chemischen Struktur als auch in Bezug auf physikalische Parameter wie beispielsweise Partikelgröße unterscheiden können und entsprechend den jeweiligen Anforderungen an den Vorratsbehälter bzw. dessen Herstellung vom Fachmann auszuwählen sind. Die Verwendung mehrerer Absorptionsmaterialien bietet den Vorteil, dass hierdurch eine breitbandige Absorption verschiedener hydrophober Substanzen erreicht werden kann, welche sich hinsichtlich ihrer chemischen oder/und physikalischen Eigenschaften (z. B. hinsichtlich ihrer Molekülgröße oder/und ihres Molekulargewichts) derart unterscheiden, dass eine selektive Absorption durch ein einziges Absorptionsmaterial nicht gewährleistet ist.

Stärker bevorzugt enthält der Vorratsbehälter ein Gemisch von mehreren Absorptionsmaterialien mit unterschiedlichen Porendurchmessern. Als besonders effektiv hat sich in diesem Zusammenhang ein Gemisch aus zwei Absorptionsmaterialien mit unterschiedlichen Porendurchmessern erwiesen, da im Innenraum des Vorratsbehälters üblicherweise eine Fülle verschiedener hydrophober, flüchtiger Substanzen zugegen ist, welche nicht allesamt identifiziert oder/und auf ihr Schadpotential hin untersucht werden können.

Die erforderlichen Mengen an Absorptionsmaterial können in Abhängigkeit von der Art des Absorptionsmaterials sowie der Art und Menge an hydrophoben, flüchtigen Substanzen in dem erfindungsgemäßen Vorratsbehälter variieren und von einem Fachmann den jeweiligen Anforderungen angepasst werden. Bringt man das zur selektiven Absorption hydrophober, flüchtiger Substanzen dienende Absorptionsmaterial beispielsweise als separates Absorberelement in den Vorratsbehälter ein, so kann die Menge an Absorptionsmaterial ohne besondere Rücksicht auf die Menge zur Herstellung des Vorratsbehälters verwendeten Materials gewählt werden. Demgegenüber enthält der Vorratsbehälter das Absorptionsmaterial im Falle eines in das Gehäuse integrierten Absorptionsmaterials üblicherweise in einer Menge von etwa 0.5 Gew.% bis etwa 25 Gew.%, insbesondere in einer Menge von etwa 1 Gew.% bis etwa 10 Gew.%, bezogen auf das Leergewicht des Vorratsbehälters.

Der erfindungsgemäße Vorratsbehälter umfasst neben dem mindestens einen Absorptionsmaterial weiterhin mindestens ein diagnostisches Element mit hydrophiler oder hydrophil beschichteter Oberfläche, welches vorzugsweise teilweise oder vollständig aus Kunststoff besteht. Somit ist es in einer Ausführungsform der Erfindung vorgesehen, dass der Vorratsbehälter mehrere diagnostische Elemente im Sinne der vorliegenden Anmeldung umfasst, wobei der Begriff "mehrere", wie er hierin verwendet wird, jegliche Zahl > 1, bevorzugt mindestens 10, stärker bevorzugt 25 oder mehr, bedeutet.

Bei dem diagnostischen Element kann es sich grundsätzlich um ein beliebiges Testelement handeln, welches für die Bestimmung des Vorhandenseins oder/und der Menge eines Analyten in einer Probe geeignet ist. Diagnostische Elemente, welche in dem erfindungsgemäßen Vorratsbehälter gelagert werden können, umfassen beispielsweise Testbänder, Teststreifen sowie die in WO 2005/084530 A2 beschriebenen Testelemente, auf welche hiermit ausdrücklich Bezug genommen wird. Die in vorliegender Anmeldung beschriebenen diagnostischen Elemente umfassen hierbei jeweils mindestens einen Testbereich, welcher mit einer den Analyten enthaltenden Probe in Kontakt gebracht werden kann und unter Verwendung geeigneter Mittel eine qualitative oder/und quantitative Bestimmung des Analyten ermöglicht.

Der Begriff "Testband", wie er hierin verwendet wird, bezeichnet ein bandförmiges Testelement, welches üblicherweise mehr als einen einzelnen Testbereich, bevorzugt mindestens 10 einzelne Testbereiche, stärker bevorzugt mindestens 25 einzelne Testbereiche, und am stärksten bevorzugt mindestens 50 einzelne Testbereiche umfasst. Vorzugsweise sind die einzelnen Testbereiche jeweils in einem Abstand von wenigen Millimetern bis zu wenigen Zentimetern, beispielsweise in einem Abstand von < 2.5 cm, voneinander angeordnet, wobei das Testband zwischen aufeinander folgenden Testbereichen gegebenenfalls Markierungsbereiche zur Wegerfassung beim Bandtransport oder/und zur Kalibrierung umfassen kann. Derartige Testbänder sind beispielsweise in EP 1 739 432 A1 beschrieben, auf deren Offenbarung ausdrücklich Bezug genommen wird.

Zur Aufnahme flüssiger, den Analyten enthaltender Proben umfassen die erfindungsgemäß eingesetzten diagnostischen Elemente vorzugsweise ein Element zur Probennahme bzw. Probennahmeelement, welches entweder direkt in das diagnostische Element integriert sein oder separat von diesem vorliegen kann. Unter einem integrierten Probennahmeelement im Sinne der vorliegenden Erfindung ist eine Vorrichtung zu verstehen, welche physikalisch mit dem diagnostischen Element verbunden ist und die aufgenommene Probe über geeignete Mittel, wie beispielsweise einen Kapillarkanal, direkt auf das Testelement transferieren kann. Ein separates Probennahmeelement ist demgegenüber als eine vom diagnostischen Element getrennt vorliegende Probennahmevorrichtung definiert, welche keine physikalische Verbindung mit dem Testelement aufweist. In diesem Fall kann ein Transfer der Probe auf das diagnostische Element beispielsweise im Anschluss an eine Remagazinierung der Probennahmevorrichtung erfolgen, wobei das diagnostische Element in dem Magazin positioniert ist.

Als Probennahmeelement kann ein beliebiges Element verwendet werden, welches eine zur Bestimmung des Analyten ausreichenden Probenmenge aufzunehmen vermag und einen nachfolgenden Transfer zumindest eines Teils der Probe auf das eigentliche Testelement, beispielsweise unter Ausnutzung von Kapillareffekten, ermöglicht. Als besonders vorteilhaft hat sich in diesem Zusammenhang die Verwendung eines Nadelelements mit Kapillarkanal erwiesen, wobei das Nadelelement vorzugsweise aus einem sterilisierbaren Material, wie beispielsweise Metall oder Kunststoff, besteht.

Vorzugsweise ist das Probennahmeelement mit einer hydrophilen Beschichtung versehen, welche bei Inkontaktbringen des Probennahmeelements mit einer den Analyten enthaltenden Probe, insbesondere einer wässrigen Probe wie Blut, benetzt wird und eine Aufnahme der Probe in das Probennahmeelement ermöglicht. Zur Bereitstellung hydrophiler Beschichtungen sind dem Fachmann eine Vielzahl von Materialien bekannt, welche vorzugsweise sowohl biokompatibel als auch sterilisierbar sind. Besonders bevorzugte Beispiele für Materialien zur Erzeugung hydrophiler Beschichtungen umfassen Polyacrylsäuren, Polyacrylate, Dextransulfat, Heparin, Lecithin und Detergenzien, sind jedoch nicht auf diese beschränkt.

Mittels des erfindungsgemäßen Vorratsbehälters besteht die Möglichkeit, diagnostische Elemente, wie oben beschrieben, für längere Zeit oder/und bei höheren Temperaturen ohne merklichen Verlust an Hydrophilie zu lagern. Insbesondere kann mittels des hierin beschriebenen Vorratsbehälters die Hydrophilie eines Probennahmeelements mit hydrophiler Beschichtung, welches in das diagnostische Element integriert oder zusammen mit diesem verpackt ist, über einen längeren Zeitraum oder/und bei erhöhten Temperaturen aufrecht erhalten werden.

Als Indikator hierfür kann beispielsweise die zur Füllung des Probennahmeelements mit der Probe benötigte Füllzeit herangezogen werden, welche sich mit zunehmender Hydrophobierung der hydrophilen Oberfläche kontinuierlich erhöht und bei handelsüblich verpackten Probennahmeelementen typischerweise bereits nach wenigen Tagen auf einen Wert von > 1 s (gemessen für ein Probennahmeelement mit Innendurchmesser 80 x 120 µm und Füllvolumen 40 nl) ansteigt. Demgegenüber weist ein erfindungsgemäß verpacktes Probennahmeelement, welches entsprechende Dimensionen besitzt, nach Lagerung für 9 Wochen bei einer Temperatur von 35°C eine deutlich geringere Füllzeit von < 1 s, bevorzugt eine Füllzeit von ≤ 0.5 s, insbesondere eine Füllzeit von ≤ 0.3 s, auf.

Alternativ besteht die Möglichkeit, die Hydrophilie eines Probennahmeelements mit hydrophiler Beschichtung über den Kontaktwinkel bzw. Benetzungswinkel zu bestimmen, welchen die hydrophil beschichtete Oberfläche des Probennahmeelements mit einem hierauf aufgebrachtem Flüssigkeitstropfen ausbildet, wobei als Flüssigkeit vorzugsweise deionisiertes Wasser zum Einsatz gelangt. Mit zunehmender Hydrophobierung der hydrophil beschichteten Oberfläche erhöht sich der Kontaktwinkel zwischen dieser Oberfläche des Probennahmeelements und einem hierauf befindlichen Wassertropfen, bis letztlich eine hydrohobe bzw. superhydrophobe Oberfläche mit einem Kontaktwinkel ≥ 90° entsteht. Insofern sieht die Anmeldung in einer bevorzugten Ausführungsform vor, dass ein erfindungsgemäß verpacktes Probennahmeelement nach Lagerung für 9 Wochen bei einer Temperatur von 35°C einen Kontaktwinkel von ≤ 40°, bevorzugt von ≤ 25°, besonders bevorzugt von ≤ 10°, mit einem auf die hydrophile Beschichtung aufgebrachten Wassertropfen ausbildet.

Die mittels des erfindungsgemäßen Vorratsbehälters gelagerten diagnostischen Elemente können zur Bestimmung einer beliebigen biologischen oder chemischen Substanz verwendet werden, welche photochemisch oder elektrochemisch nachweisbar ist. Vorzugsweise wird der Analyt aus der Gruppe bestehend aus Äpfelsäure, Alkohol, Ammonium, Ascorbinsäure, Cholesterin, Cystein, Glucose, Glutathion, Glycerin, Harnstoff, 3-Hydroxybutyrat, Milchsäure, 5'-Nukleotidase, Peptiden, Pyruvat, Salicylat und Triglyceriden ausgewählt, wobei Glucose besonders bevorzugt ist. Der Analyt kann hierbei aus einer beliebigen Quelle stammen, ist jedoch bevorzugt in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, enthalten. Bevorzugt wird mittels der hierin beschriebenen diagnostischen Elemente das Vorhandensein oder/und die Menge eines Analyten in einer Probe aus Vollblut, Plasma, Serum oder extrazellulärer Gewebeflüssigkeit bestimmt.

In einer Variante der vorliegenden Erfindung kann der erfindungsgemäße Vorratsbehälter neben dem mindestens einen diagnostischen Element mit hydrophiler oder hydrophil beschichteter Oberfläche und dem mindestens einen Absorptionsmaterial zur selektiven Absorption hydrophober, flüchtiger Substanzen zusätzlich mindestens ein Trocknungsmittel, d. h. ein Absorptionsmaterial zur selektiven Absorption von Wasserdampf, umfassen, wodurch eine längere Haltbarkeit feuchtigkeitsempfindlicher Substanzen der hierin beschriebenen diagnostischen Elemente, wie beispielsweise Enzyme oder/und Coenzyme, erzielt werden kann. Absorptionsmaterialien, welche zur selektiven Absorption von Wasserdampf geeignet sind, sind dem Fachmann allgemein bekannt und umfassen beispielsweise Kieselgel und kleinporige Zeolithe, wobei der Ausdruck "Absorptionsmaterial zur selektiven Absorption von Wasserdampf' jegliches Material bezeichnet, das bei den jeweiligen Umgebungsbedingungen eine höhere Affinität für Wasser aufweist als für eine hydrophobe, flüchtige Substanz im Sinne der vorliegenden Anmeldung.

In einer bevorzugten Variante ist der erfindungsgemäße Vorratsbehälter allerdings frei von Trocknungsmitteln. Somit ist es mittels des hierin beschriebenen Vorratsbehälters möglich, diagnostische Elemente mit hydrophiler oder hydrophil beschichteter Oberfläche auch ohne Trocknungsmittel für eine längere Zeit, beispielsweise für eine Dauer von mindestens 2 Wochen, vorzugsweise von mindestens 4 Wochen und besonders bevorzugt von mindestens 8 Wochen, oder/und bei erhöhten Temperaturen, beispielsweise bei einer Temperatur von mindestens 20°C, vorzugsweise von mindestens 25°C und besonders bevorzugt von mindestens 30°C, zu lagern, ohne dass es infolge Sättigung des Absorptionsmaterials mit Wasser zu Ablagerungen auf der hydrophilen oder hydrophil beschichteten Oberfläche der diagnostischen Elemente kommt.

In einer bevorzugten Ausführungsform enthält der erfindungsgemäße Vorratsbehälter das mindestens eine diagnostische Element steril verpackt. Zu diesem Zweck kann ein zu sterilisierendes diagnostisches Element insbesondere vor Durchführung der Sterilisation in einen hierin beschriebenen Vorratsbehälter eingebracht werden, woraufhin der Vorratsbehälter verschlossen und das diagnostische Element in dem verschlossenen Vorratsbehälter sterilisiert werden kann. Die sterile Verpackung ermöglicht es, das diagnostische Element bis zu seinem späteren Gebrauch steril zu halten, ohne dass es einer erneuten Sterilisation bedarf. Besonders bevorzugt sieht die Erfindung demnach vor, dass es sich bei dem diagnostischen Element um einen Einwegartikel handelt, welcher nach Gebrauch infolge Verlusts der Sterilität nicht erneut verwendet wird.

Die Sterilisation der diagnostischen Elemente kann auf unterschiedliche Weise erfolgen und umfasst vorzugsweise ionisierende Strahlung, wie beispielsweise Elektronenstrahlung oder/und Gammastrahlung. Da ionisierende Strahlung andererseits häufig eine Schädigung der zur Herstellung von Vorratsbehältern üblicherweise verwendeten Materialien nach sich zieht, in deren Folge wiederum hydrophobe, flüchtige Substanzen erzeugt und freigesetzt werden können, besteht ein besonderer Vorteil der erfindungsgemäßen Vorratsbehälter in ihrer Unempfindlichkeit gegenüber sterilisationsbedingter Materialschädigung bzw. im Schutz darin gelagerter diagnostischer Elemente vor sterilisationsbedingter Hydrophobierung.

Die hierin beschriebenen Vorratsbehälter können grundsätzlich aus einem beliebigen Material bestehen, welches für die Zwecke einer Lagerung diagnostischer Elemente mit hydrophiler oder hydrophil beschichteter Oberfläche geeignet erscheint. In einer bevorzugten Ausführungsform der Erfindung ist der Vorratsbehälter zumindest teilweise, d. h. teilweise oder vollständig, aus einem mindestens eine hydrophobe, flüchtige Substanz im Sinne der vorliegenden Anmeldung umfassenden Material gebildet, wobei u. a. Kunststoffe und Papier in Frage kommen. Stärker bevorzugt handelt es sich bei dem mindestens eine hydrophobe, flüchtige Substanz umfassenden Material um einen Kunststoff, wobei insbesondere Kunststoffe auf Basis von Polyamid, Polycarbonat, Polyester, Polyethylen oder Polypropylen zur Anwendung gelangen können.

Die erfindungsgemäßen Vorratsbehälter umfassen grundsätzlich jede dem Fachmann geläufige und für die Zwecke der vorliegenden Erfindung geeignet erscheinende physikalische Form, sofern sie die Aufnahme mindestens eines diagnostischen Elements, insbesondere eines Testbandes oder Teststreifens, gestatten. Bevorzugte Vorratsbehälter im Sinne der vorliegenden Erfindung umfassen insbesondere Blistermagazine, Leporellomagazine, Scheibenmagazine, Stapelmagazine und Trommelmagazine, welche beispielsweise in EP 0 951 939 A2, EP 1 022 565 A2, EP 1 736 772 A1 und WO 2005/104948 A1 beschrieben sind. Auf die Offenbarung vorstehender Dokumente, insbesondere hinsichtlich der Geometrie der Vorratsbehälter, wird hiermit ausdrücklich Bezug genommen.

In einem weiteren Aspekt betrifft die Erfindung ein analytisches Messgerät, welches einen erfindungsgemäßen Vorratsbehälter umfasst und zur qualitativen oder/und quantitativen Bestimmung eines Analyten dient. Beispiele für derartige Messgeräte umfassen u. a. die kommerziell erhältlichen Produkte Accu-Chek® Active, Accu-Chek® Compact und Accu-Chek® Mobile (alle Fa. Roche), sind jedoch nicht auf diese beschränkt.

In noch einem weiteren Aspekt betrifft die Erfindung die Verwendung eines Absorptionsmaterials zur selektiven Absorption hydrophober, flüchtiger Substanzen in einem Vorratsbehälter, wobei der Vorratsbehälter mindestens ein diagnostisches Element mit hydrophiler oder hydrophil beschichteter Oberfläche umfasst. Was die Ausgestaltung des Absorptionsmaterials anbetrifft, so wird auf die Ausführungen in Zusammenhang mit der Beschreibung des erfindungsgemäßen Vorratsbehälters verwiesen.

Die Erfindung soll durch die nachfolgenden Beispiele näher erläutert werden.

### Beispiele

### Beispiel 1: Hydrophobierung hydrophil beschichteter Oberflächen durch polymere Verpackungsmaterialien

Um den Einfluss hydrophober, aus polymeren Verpackungsmaterialien austretender Substanzen auf die Füllzeit diagnostisch verwendeter Probennahmeelemente zu bestimmen, wurden Mikrokapillaren mit einem Innendurchmesser von 80 x 120 µm und einem Füllvolumen von 40 nl (bei 4 mm Länge), welche eine hydrophile Beschichtung aus Heparin aufwiesen, zusammen mit 7.0 g verschiedener handelsüblich erhältlicher Kunststoffe für jeweils 9 Wochen bei 35°C gelagert. Anschließend wurden die Mikrokapillaren mit einer antikoagulierten Venenblutprobe in Kontakt gebracht und die zum Erreichen einer Füllhöhe von 4 mm benötigte Zeit in Sekunden bestimmt (n = 8). Die Ergebnisse sind in Tabelle 1 dargestellt.bedeutet in diesem Zusammenhang keine Füllung.

**Tabelle 1**

| **Kunststoff** | **Füllzeit nach 9 Wochen bei 35°C [s/4 mm]** | | | |
|---|---|---|---|---|
| | | | | |
| PET-Folie pur ohne Betastrahlung (Vergleich) | 0.2 | 0.2 | 0.2 | 0.3 |
| | 0.3 | 0.3 | 0.3 | 0.2 |
| PET-Folie pur (Vergleich) | 0.2 | 0.3 | 0.3 | 0.2 |
| | 0.2 | 0.2 | 0.3 | 0.3 |
| Rynite 415HP natur | --- | --- | --- | --- |
| | --- | --- | --- | --- |
| Rynite 530NC101 natur | 0.4 | 0.8 | 1.1 | 1.1 |
| | --- | 0.8 | --- | 1.9 |
| Impet 2700GV 1/20 natur | 1.3 | --- | --- | --- |
| | 1.7 | --- | --- | --- |
| Crastin S600F40NC natur | 0.3 | 0.2 | 0.3 | 0.2 |
| | 0.3 | 0.2 | 0.3 | 0.3 |
| Polyester RT6012 transparent | --- | --- | --- | --- |
| | --- | --- | --- | --- |
| Trogamid CX9704 | 1.4 | --- | 0.8 | --- |
| | --- | --- | 1.3 | 1.1 |
| Novodur P2H-AT natur | --- | --- | --- | --- |
| | --- | --- | --- | --- |
| Novodur P2H-AT schwarz | --- | --- | --- | --- |
| | --- | --- | --- | --- |
| Makrolon 2405 glasklar | 0.6 | 0.3 | 0.3 | 0.3 |
| | 0.5 | 0.3 | 0.3 | 0.3 |
| Lustran H604 schwarz | --- | --- | --- | --- |
| | --- | --- | --- | --- |
| Terlux KR2812 | --- | --- | --- | --- |
| | --- | --- | --- | --- |
| Finalloy EBC-UC 142UC9B9 | --- | --- | --- | --- |
| | --- | --- | --- | --- |
| Ticona PET Reinpolymer | --- | --- | --- | --- |
| | --- | --- | --- | --- |
| Alu-Hotmelt 115-5018 | 0.5 | 0.5 | 0.6 | 0.4 |
| | 0.5 | 0.5 | 0.4 | 0.4 |
| Alu-Hotmelt 112-0180 | --- | --- | --- | --- |
| | --- | --- | --- | --- |
| Alu-Hotmelt 112-0433 | --- | --- | --- | --- |
| | --- | --- | --- | --- |
| PES Ultrason E1010 natur | 0.4 | 0.5 | 0.5 | 0.4 |
| | 1.9 | 0.4 | 0.4 | 0.4 |
| SAN Luran 358N natur | 1.2 | 0.7 | 0.6 | --- |
| | --- | 0.8 | --- | 0.5 |
| PS Polystyrol 145D | 2.4 | --- | 1.5 | 0.8 |
| | --- | 2.2 | 1.1 | 1.0 |
| Zeonor 1060 | 0.4 | 0.4 | 0.7 | 0.8 |
| | 0.9 | 0.5 | 0.6 | 0.6 |
| Celanex CX2002 natur | 0.7 | 0.8 | --- | 1.1 |
| | 0.6 | 1.1 | --- | 1.0 |
| Celanex CX2003 natur | 0.8 | --- | 0.5 | 0.6 |
| | --- | 0.8 | 0.6 | 0.5 |
| Ultradur S4090 G4 | 0.6 | --- | 0.8 | 0.7 |
| | --- | 1.3 | 0.5 | --- |

Wie aus Tabelle 1 hervorgeht, verlängert sich die Füllzeit der Mikrokapillaren nach Lagerung in Gegenwart der meisten handelsüblichen Kunststoffe signifikant bzw. führt in vielen Fällen zu einem vollständigen Verlust der Aufnahmefähigkeit für Probenmaterial, so dass diese Kunststoffe als Verpackungsmaterial für diagnostische Elemente ohne zusätzliche Schutzmaßnahmen nicht oder nur bedingt einsetzbar sind. Vielmehr gewährleisten lediglich PET, Crastin® S600F40NC und Makrolon® 2405 nach entsprechender Lagerung der Mikrokapillaren noch eine Füllzeit von < 0.5 s, was auf eine geringe Menge an hydrophoben, flüchtigen Substanzen in diesen kommerziell erhältlichen Polymeren rückschließen lässt.

### Beispiel 2: Reduktion der durch flüchtige Substanzen aus polymeren Verpackungsmaterialien vermittelten Hydrophobierung hydrophil beschichteter Oberflächen

Um den Einfluss verschiedener, der Absorption hydrophober, flüchtiger Substanzen dienender Materialien auf die Hydrophobierung hydrophil beschichteter Probennahmeelemente zu untersuchen, wurden Mikrokapillaren gemäß Beispiel 1, welche mittels Elektronenstrahlung sterilisiert worden waren, für 9 Wochen bei 35°C in Gegenwart von 7 g Novodur® P2H-AT natur und ggf. 1 g Absorptionsmaterial in einem PET-Beutel gelagert. Die Ergebnisse sind in Tabelle 2 dargestellt.

**Tabelle 2**

| **Probe** | **Füllzeit nach 9 Wochen bei 35°C [s/4 mm]** |
|---|---|
| | |
| Mikrokapillare | 0.24 |
| Mikrokapillare + Elektronenstrahlung | 0.22 |
| Mikrokapillare + Elektronenstrahlung + Novodur® | keine Füllung |
| Mikrokapillare + Elektronenstrahlung + Novodur® + ZEOflair® 100 | 1.15 |
| Mikrokapillare + Elektronenstrahlung + Novodur® + ZEOflair® 200 | 0.48 |
| Mikrokapillare + Elektronenstrahlung + Novodur® + ZEOflair® 300 | 0.21 |

Wie aus Tabelle 2 ersichtlich ist, führt die Sterilisation und Lagerung in "sauberer" Verpackung (PET-Beutel) nicht zu einem Verlust der Aufnahmefähigkeit für Probenmaterial. Dagegen wird im Falle einer Sterilisation und Lagerung der Mikrokapillaren in Gegenwart von Novodur® P2H-AT natur bei Abwesenheit eines Absorptionsmaterials ein vollständiger Verlust der Aufnahmefähigkeit für Probenmaterial beobachtet (siehe auch Tabelle 1).

Durch Hinzufügen von ZEOflair® 100 (Porengröße 0.56 nm) als Absorptionsmaterial kann einer Hydrophobierung der Mikrokapillaren teilweise entgegen gewirkt werden, wobei eine Füllzeit von 1.15 s nach 9 Wochen Lagerung bei 35°C dennoch auf eine deutliche Hydrophobierung der hydrophil beschichteten Oberfläche durch hydrophobe, aus dem Kunststoff austretende Substanzen schließen lässt.

Eine signifikante Erhöhung der Schutzwirkung für hydrophil beschichtete Mikrokapillaren lässt sich durch Verwendung von ZEOflair® 200 (Porengröße 0.76 nm) als Absorptionsmaterial erzielen, wie eine Füllzeit von 0.48 s belegt. Bei Verwendung von ZEOflair® 300 (Gemisch aus ZEOflair® 100 und ZEOflair® 200) als Absorptionsmaterial wird schließlich eine optimale Absorption der aus Novodur® P2H-AT natur ausdunstenden hydrophoben Substanzen erreicht, wie sich aus einer Füllzeit von 0.21 s nach 9 Wochen Lagerung bei 35°C ergibt. Die hydrophoben, flüchtigen Substanzen weisen offensichtlich ein uneinheitliches Molekulargewicht auf, weshalb das eingesetzte Zeolithgemisch besonders vorteilhafte Ergebnisse liefert.

### Beispiel 3: Reduktion der durch flüchtige Substanzen aus Testelementen vermittelten Hydrophobierung hydrophil beschichteter Oberflächen

Um den Einfluss der Testchemie herkömmlich verwendeter diagnostischer Elemente auf die Hydrophobierung hydrophil beschichteter Probennahmeelemente zu untersuchen, wurden Mikrokapillaren gemäß Beispiel 1 für 9 Wochen bei 35°C in Gegenwart von 0.92 g Trägermaterial bzw. in Gegenwart zweier unterschiedlicher, zur Herstellung von Teststreifen verwendeter Zusammensetzungen (jeweils 1.00 g Reaktionsfilm) unter verschiedenen Bedingungen in PET-Beuteln gelagert. Die Ergebnisse sind in Tabelle 3 dargestellt.

**Tabelle 3**

| **Probe** | **Füllzeit nach 9 Wochen bei 35°C [s/4 mm]** |
|---|---|
| | |
| Mikrokapillare (Vergleich) | 0.235 |
| Mikrokapillare + Pokalonfolie 140 µm | 0.300 |
| Mikrokapillare + Accu-Chek Active Chemiebeschichtung | keine Füllung |
| Mikrokapillare + Accu-Chek Active Chemiebeschichtung (vorab 3 Wochen Lagerung bei 35°C in Gegenwart von ZEOflair® 300) | keine Füllung |
| Mikrokapillare + Accu-Chek Active Chemiebeschichtung + ZEOflair® 300 | 0.208 |

Gemäß Tabelle 3 führt die Lagerung der Mikrokapillaren in Gegenwart von Trägermaterial (Pokalonfolie) zu einer leichten Hydrophobierung der hydrophil beschichteten Mikrokapillaren, wie sich aus einer Erhöhung der Füllzeit von 0.235 s (Vergleich) auf 0.300 s ableiten lässt.

Bei Lagerung der Mikrokapillaren in Gegenwart einer SC-V-Chemiebeschichtung, wie sie u.a. in den kommerziell erhältlichen Testelementen Accu-Chek® Active, Accu-Chek® Compact oder Accu-Chek® Mobile (alle Fa. Roche) Anwendung findet, wurde demgegenüber infolge starker Hydrophobierung der hydrophil beschichteten Mikrokapillaren ein vollständiger Verlust der Aufnahmefähigkeit für Probenmaterial festgestellt, welcher auch durch eine vorherige dreiwöchige Lagerung der Testchemie in Gegenwart von ZEOflair® 300 nicht verhindert werden konnte. Im Falle einer Lagerung der Mikrokapillaren in Gegenwart der Testchemie und ZEOflair® 300 als Absorptionsmaterial konnte eine Schädigung bzw. Hydrophobierung der Mikrokapillaren vollständig vermieden werden.

## Patentansprüche

1. Vorratsbehälter, umfassend
(a) mindestens ein diagnostisches Element mit hydrophiler oder hydrophil beschichteter Oberfläche, und
(b) mindestens ein porenhaltiges Absorptionsmaterial zur selektiven Einlagerung hydrophober, flüchtiger Substanzen,
**dadurch gekennzeichnet,**
**dass** das Absorptionsmaterial Poren mit einem Durchmesser von etwa 0,5 nm bis etwa 0,8 nm aufweist.

2. Vorratsbehälter nach Anspruch 1,
**dadurch gekennzeichnet,**
**dass** das Absorptionsmaterial ein natürlich vorkommendes oder synthetisches Silikat, insbesondere ein Zeolith, ist.

3. Vorratsbehälter nach Anspruch 1 oder 2,
**dadurch gekennzeichnet,**
**dass** er das Absorptionsmaterial in Form eines separaten Absorberelements oder/und in seinem Gehäuse integriert enthält.

4. Vorratsbehälter nach einem der Ansprüche 1 bis 3,
**dadurch gekennzeichnet,**
**dass** er ein Gemisch aus mehreren Absorptionsmaterialien, insbesondere ein Gemisch aus mehreren Absorptionsmaterialien mit unterschiedlichen Porendurchmessern, umfasst.

5. Vorratsbehälter nach einem der Ansprüche 1 bis 4,
**dadurch gekennzeichnet,**
**dass** das diagnostische Element ein Probennahmeelement, insbesondere ein Nadelelement mit Kapillarkanal, separat oder integriert umfasst.

6. Vorratsbehälter nach Anspruch 5,
**dadurch gekennzeichnet,**
**dass** das Probennahmeelement mit einer hydrophilen Beschichtung, insbesondere einer Beschichtung aus Polyacrylsäure, Polyacrylat, Dextransulfat, Heparin, Lecithin oder einem Detergenz, versehen ist.

7. Vorratsbehälter nach Anspruch 6,
**dadurch gekennzeichnet,**
**dass** das Probennahmeelement nach Lagerung für 9 Wochen bei einer Temperatur von 35°C einen Kontaktwinkel von ≤ 40°, insbesondere von ≤ 25°, mit einem auf die hydrophile Beschichtung aufgebrachten Wassertropfen ausbildet.

8. Vorratsbehälter nach einem der Ansprüche 1 bis 7,
**dadurch gekennzeichnet,**
**dass** das diagnostische Element als Testband oder Teststreifen ausgebildet ist.

9. Vorratsbehälter nach einem der Ansprüche 1 bis 8,
**dadurch gekennzeichnet,**
**dass** er das diagnostische Element steril verpackt enthält.

10. Vorratsbehälter nach einem der Ansprüche 1 bis 9,
**dadurch gekennzeichnet,**
**dass** er frei von Trocknungsmitteln ist.

11. Vorratsbehälter nach einem der Ansprüche 1 bis 10,
**dadurch gekennzeichnet,**
**dass** er zumindest teilweise aus einem mindestens eine hydrophobe, flüchtige Substanz umfassenden Material, insbesondere aus einem Kunststoff auf Basis von Polyamid, Polycarbonat, Polyester, Polyethylen oder Polypropylen, gebildet ist.

12. Vorratsbehälter nach einem der Ansprüche 1 bis 11,
**dadurch gekennzeichnet,**
**dass** er als Blistermagazin, Leporellomagazin, Scheibenmagazin, Stapelmagazin oder Trommelmagazin ausgebildet ist.

13. Analytisches Messgerät, umfassend einen Vorratsbehälter nach einem der Ansprüche 1 bis 12.

14. Verwendung eines Vorratsbehälters nach einem der Ansprüche 1-12 zur selektiven Einlagerung hydrophober, flüchtiger Substanzen in dem Vorratsbehälter, wobei der Vorratsbehälter mindestens ein diagnostisches Element mit hydrophiler oder hydrophil beschichteter Oberfläche umfasst.

## Claims

1. Storage container comprising
(a) at least one diagnostic element with a hydrophilic or hydrophilically coated surface, and
(b) at least one porous absorption material for the selective storage of hydrophobic, volatile substances,
**characterised in that**
the absorption material has pores with a diameter of some 0.5 nm to some 0.8 nm.

2. Storage container in accordance with claim 1,
**characterised in that**
the absorption material is a naturally occurring or synthetic silicate, in particular a zeolite.

3. Storage container in accordance with claim 1 or 2,
**characterised in that**
it contains the absorption material in the form of a separate absorber element or/and integrated into its housing.

4. Storage container in accordance with one of claims 1 to 3,
**characterised in that**
it comprises a mixture of several absorption materials, in particular a mixture of several absorption materials with different pore diameters.

5. Storage container in accordance with one of claims 1 to 4,
**characterised in that**
the diagnostic element comprises a separate or integrated sampling element, in particular a needle element with a capillary duct.

6. Storage container in accordance with claim 9,
**characterised in that**
the sampling element is equipped with a hydrophilic coating, in particular a coating made of polyacrylic acid, polyacrylate, dextran sulphate, heparin, lecithin or a detergent.

7. Storage container in accordance with claim 6,
**characterised in that**
after storage for 9 weeks at a temperature of 35°C, the sampling element forms a contact angle of ≤ 40°, in particular of ≤ 25°, with a water drop deposited on the hydrophilic coating.

8. Storage container in accordance with one of claims 1 to 7,
**characterised in that**
the diagnostic element is designed as a test tape or test strip.

9. Storage container in accordance with one of claims 1 to 8,
**characterised in that**
it contains the sterilely packaged diagnostic element.

10. Storage container in accordance with one of claims 1 to 9,
**characterised in that**
it is free of desiccants.

11. Storage container in accordance with one of claims 1 to 10,
**characterised in that**
it is formed to some extent at least of a material comprising a hydrophobic, volatile substance, made in particular from a plastic based on polyamide, polycarbonate, polyester, polyethylene or polypropylene.

12. Storage container in accordance with one of claims 1 to 11,
**characterised in that**
it is designed as a blister magazine, fanfold magazine, disc magazine, stack magazine or drum magazine.

13. Analytical measuring device comprising a storage container in accordance with one of claims 1 to 12.

14. Use of a storage container in accordance with one of claims 1-12 for the selective storage of hydrophobic, volatile substances in the storage container, in which the storage container comprises at least one diagnostic element with a hydrophilic or hydrophilically coated.

## Revendications

1. Réservoir, comprenant
(a) au moins un élément diagnostique comportant une surface qui est hydrophile ou pourvue d'un revêtement hydrophile, et
(b) au moins un matériau d'absorption contenant des pores, destiné au dépôt sélectif de substances volatiles hydrophobes,
**caractérisé en ce**
**que** ledit matériau d'absorption comporte des pores ayant un diamètre compris entre environ 0,5 nm et environ 0,8 nm.

2. Réservoir selon la revendication 1,
**caractérisé en ce**
**que** ledit matériau s'absorption est un silicate naturel ou synthétique, notamment une zéolithe.

3. Réservoir selon les revendication 1 ou 2,
**caractérisé en ce**
**qu'**il contient ledit matériau d'absorption sous forme d'un élément d'absorption séparé ou/et de manière à ce que celui-ci fasse partie intégrante de son boîtier.

4. Réservoir selon l'une des revendications 1 à 3,
**caractérisé en ce**
**qu'**il comprend un mélange de plusieurs matériaux d'absorption, notamment un mélange de plusieurs matériaux d'absorption ayant des diamètres de pores différents.

5. Réservoir selon l'une des revendications 1 à 4,
**caractérisé en ce**
**que** ledit élément diagnostique comprend, de manière séparée ou intégrée, un élément de prélèvement d'échantillons, notamment un élément d'aiguille comportant un canal capillaire.

6. Réservoir selon la revendication 5,
**caractérisé en ce que**
ledit élément de prélèvement d'échantillons est pourvu d'un revêtement hydrophile, notamment d'un revêtement en acide polyacrylique, polyacrylate, sulfate de dextrane, héparine, lécithine ou en un détergent.

7. Réservoir selon la revendication 6,
**caractérisé en ce**
**que** ledit élément de prélèvement d'échantillons présente, suite à un stockage de 9 semaines à une température de 35 °C, un angle de contact de ≤ 40°, notamment de ≤ 25°, avec une goutte d'eau qu'on applique sur ledit revêtement hydrophile.

8. Réservoir selon l'une des revendications 1 à 7,
**caractérisé en ce**
**que** ledit élément diagnostique est réalisé sous forme d'un ruban d'essai ou d'une bandelette d'essai.

9. Réservoir selon l'une des revendications 1 à 8,
**caractérisé en ce**
**qu'**il contient ledit élément diagnostique dans un conditionnement stérile.

10. Réservoir selon l'une des revendications 1 à 9,
**caractérisé en ce**
**qu'**il est exempt de agents de déshydratation.

11. Réservoir selon l'une des revendications 1 à 10,
**caractérisé en ce**
**qu'**il est au moins partiellement réalisé en au moins un matériau comprenant au moins une substance volatile hydrophobe, notamment en une matière plastique à base de polyamide, polycarbonate, polyester, polyéthylène ou polypropylène.

12. Réservoir selon l'une des revendications 1 à 11,
**caractérisé en ce**
**qu'**il est réalisé sous forme de dispositif de stockage pour blisters, dispositif de stockage en accordéon, dispositif de stockage pour disques, dispositif de stockage à empilement ou dispositif de stockage en tambour.

13. Appareil de mesure analytique, comprenant un réservoir selon l'une des revendications 1 à 12.

14. Utilisation d'un réservoir selon l'une des revendications 1 à 12 pour le dépôt sélectif de substances volatiles hydrophobes dans ledit réservoir, ledit réservoir comprenant au moins un élément diagnostique comportant une surface qui est hydrophile ou pourvue d'un revêtement hydrophile.
